# EUROPÄISCHE PATENTSCHRIFT

(11) **EP 0 562 069 B1**
(45) Veröffentlichungstag und Bekanntmachung des Hinweises auf die Patenterteilung: **19.06.1996**
(21) Anmeldenummer: 92919872.9
(22) Anmeldetag: 02.10.1992
(51) Int. Cl.: C07D 307/64

(54) **VERFAHREN ZUR HERSTELLUNG VON 3-S-FURANDERIVATEN**
METHOD OF PREPARING 3-(S)-FURAN DERIVATIVES
PROCEDE DE FABRICATION DE DERIVES DE FURANNE 3-S

(30) Priorität: 11.10.1991 CH 2994/91
(43) Veröffentlichungstag der Anmeldung: 29.09.1993
(73) Patentinhaber: GIVAUDAN-ROURE (INTERNATIONAL) S.A., CH-1214 Vernier, Genève (CH)
(72) Erfinder: HUBER, Ulrich, CH-8032 Zürich (CH)
(74) Vertreter: Urech, Peter, Dr.
(86) Internationale Anmeldenummer: CH9200199
(87) Internationale Veröffentlichungsnummer: WO9307134

(56) Entgegenhaltungen:
- GB-A- 1 543 653
- US-A- 3 922 288
- US-A- 4 020 175
- JOURNAL OF HETEROCYLIC CHEMISTRY Vol. 14 1977,pages 281-288 S.GRONOWITZ ET AL.:"Inverted Reactivity of Aryllithium Derivates V. on Sybthesis of Thiocyano-Phenylsulfinyl-, and Phenoxy Derivatives of Thiophenes and Furans see page284;table 2 see page 286 left hand collum, last paragraph X
- V.JäGER ET AL.:UMWANDLUNG VON ALKINEN IN :"Houben- Weyl: methoden der Organischen chemie, 4 Aufl. Vol. V/2a, paragraph B,II, pages 687-768 " 8 September 1977, GEOG THIEME VERLAG; STUTTGART; SEE pages 743-751,in particular page 747
- HELVETICA CHIMICA ACTA. Vol. 72,1989, BASEL CH pages 477-456 D.OLBRECHT "Acid-Catalyzed Cyyclization Reactions of Substituted Acetylenic ketones: A approch for the Synthesis of 3-Halofurans, Flavones, and Styrylchromones see shems 1,4 to5

## Beschreibung

Die Erfindung betrifft ein Verfahren zur Herstellung von substituierten Furanen, welche insbesondere Aromastoffe darstellen; dabei werden 4-Hydroxy-2-in-1-one oder Acetale oder Ketale davon mit nukleophilen S-Verbindungen zu 3-S-Furanen cyclisiert, wobei dieses 3-S-Atom beliebig substituiert sein kann, und die Acetylenderivate, die 4-Hydroxy-2-in-1-one, gegebenenfalls 1- und/oder 4-alkyl- oder alkenyl substituiert sein können.

Genauer gesagt, betrifft die Erfindung ein Verfahren zur Herstellung von substituierten Furanderivaten der Formel 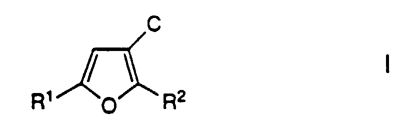
worin R¹ und R² bedeuten: Wasserstoff, C₁₋₆-Alkyl, wie CH₃, C₂H₅, C₂₋₆-Akenyl, wie Propenyl, etc.
und C einen schwefelhaltigen Rest darstellt.
Das Verfahren ist dadurch gekennzeichnet, dass man ein Acetylenderivat 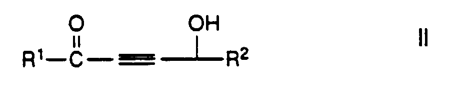 oder ein Acetal oder Ketal davon, mittels einer, mindestens ein nukleophiles Schwefelatom enthaltenen Verbindung

AB III

worin
A = H⁺, NH₄⁺, N(C₁₋₆-Alkyl)₃H⁺, ein Alkalimetallkation, ein Erdalkalimetallkation,
B = R⁴-S⁻, SCN⁻, AS₂O₃⁻, H₂NC(=S)NH⁻, R⁵-S(=O)O⁻, R⁵-S-S⁻, und
R⁴ = H, C₁₋₆-Alkyl, z.B. Methyl, sec.Butyl,
   C₂₋₆-Alkenyl, z.B. Propenyl, 2-Methyl-butenyl, etc.,
   C₂₋₆-Alkinyl, z.B. Butynyl (1), Propynyl (1), durch Halogen (F, Cl, Br, J), OH, SH, C=O oder einen C₁₋₆-Carbonsäurerest oder ein C₁₋₆-Alkylcarbonsäurederivat, z.B. -CH₂(CH₂)₂COOC₂H₅, -CH₂CH(CH₃)CH₂COCH₃, -CH₂CH(CH₃)SH, substituiertes C₁₋₆-Alkyl,
   C₁₋₆-Acyl, z.B. C₁₋₆-Akanoyl, wie Acetyl, Butyryl, (beliebig, auch heterocyclisch) substituiertes C₁₋₆-Acyl, z.B. 2-Tetrahydrofuranylmethylcarbonyl,
R⁵ = C₁₋₆-Alkyl, z.B. CH₃, Aryl, z.B. Phenyl, Heteroaryl, insbesondere Reste von 5-Ring-Verbindungen, wie Furyl oder Thiophenyl,
cyclisiert, und das primäre Reaktionsprodukt der Formel 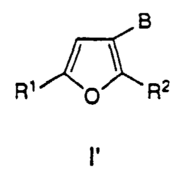 gegebenenfalls durch eine Sekundärreaktion in ein Folgeprodukt der Formel 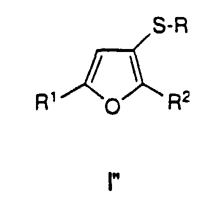
worin R = R⁴; oder SR⁵, z.B. S-C₁₋₆-Alkyl, wie S-CH₃, S-sec.Butyl, etc. mit Halogen, Hydroxy, SH, Carbonyl, Carboxyl funktionalisiertes S-C₁₋₆-Akyl, S-Aryl, wie Thio-phenyl, S-Heteroaryl, wie Thio-furfuryl, 3-S-Thio-furyl, 3-S-Thiophenyl,
wobei alle im Rahmen der vorliegenden Erfindung vorkommenden (Hetero)Aryle beliebig durch R¹ und R² substituiert sein können, und alle Kohlenwasserstoffreste geradkettig oder verzweigt sein können,
überführt.

Das Verfahren kann wie folgt schematisch dargestellt werden:

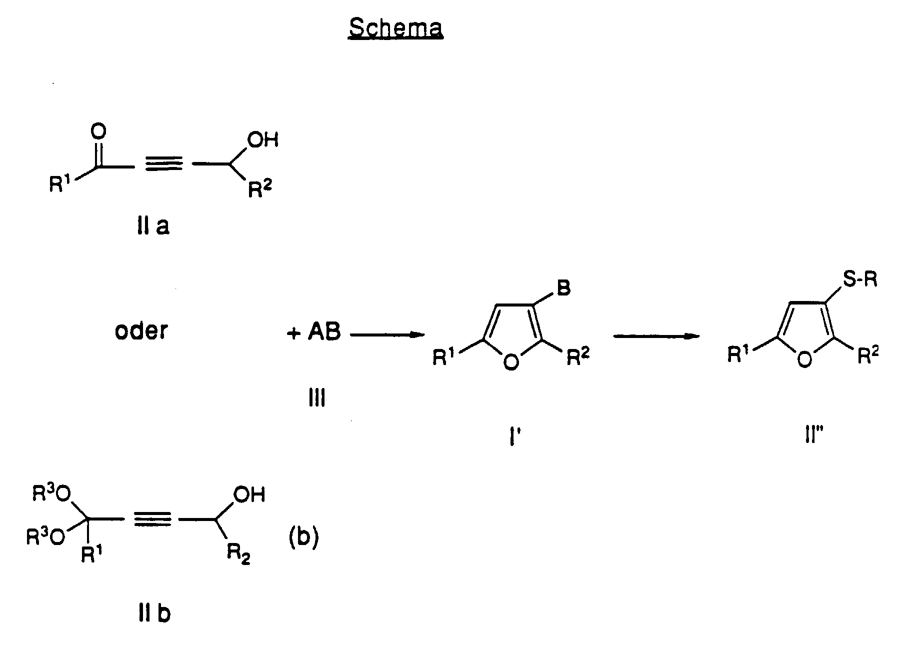

Die Ausgangsmaterialien II und III werden zweckmässigerweise im Verhältnis von ca. 2:1 bis ca. 1:5, bevorzugt ca. 1:1, unter Zugabe einer Mineralsäure, wie Salpetersäure, Phosphorsäure oder, vorzugsweise Schwefelsäure, oder auch einer organischen Säure, z.B. Essigsäure, Zitronensäure, etc. und eines organischen Lösungsmittels, wie eines aliphatischen oder aromatischen, gegebenenfalls halogenierten Kohlenwasserstoffes, Aethers, Alkohols, etc. wie Pentan, Methylenchlorid, Toluol, Diäthyläther, Tetrahydrofuran, MTBE, Aethanol, etc. zusammengegeben und bei Temperaturen von ca. 0° bis ca. 100°C, vorzugsweise ca. 20-50°C gerührt.

Vorzugsweise wird dabei II zu III zugetropft und bei pH-Werten unter ca. 4 gearbeitet. In gewissen Fällen wurde beobachtet, dass vorgängige Basenbehandlung des Gemisches der Ausgangsmaterialien II + III die Addition von B in β-Stellung von II beschleunigt. Als Basen können hierbei vorzugsweise tertiäre Amine, wie z.B. N(C₂H₅)₃, Diazabicyclooctan, p-Diaminopyridin, etc. fungieren. Die geeigneten Temperaturen sind dabei ca. 20° bis ca. 50°C. Diese Vorgehensweise ist insbesondere bei AB = R⁴SH, R⁵-S-S⁻H, etc. angezeigt.

Die Reaktion von II mit III kann ein- oder zweiphasig (polar/apolar) durchgeführt werden. Letztere Variante ist bevorzugt. Im Falle der ersteren Variante arbeitet man bevorzugt in polaren Lösungsmitteln, wie Alkoholen, z.B. Aethanol, etc., Aceton, Acetonitril, Wasser, etc. oder Gemischen solcher Lösungsmittel.

Die Acetalisierung bzw. Ketalisierung von II erfolgt in an sich bekannter Weise, und es bedeuten in der Formel II(b): 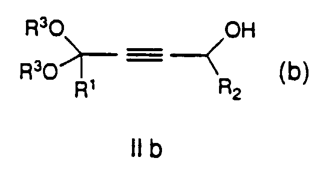
R³ = C₁₋₄-Alkyl
R³ + R³ + C von IIb = 5- oder 6-gliedriger Ring.

Das Radikal B der Verbindung I' kann Folgereaktionen unterworfen werden, wobei die Verbindung I" entsteht. Die Art der Folgereaktion hängt insbesondere von der Natur von B ab.

Für die Sekundärreaktionen I' → I" gilt deshalb allgemein folgendes: Die untenstehenden Reaktionen α) bis δ) sind dem Fachmann wohlbekannte Standardreaktionen, und die aufgeführten Parameter, wie Reagenzien, Reaktionsbedingungen etc. sind deshalb als rein illustrierend anzusehen.

### a) Hydrolyse resp. Alkoholyse

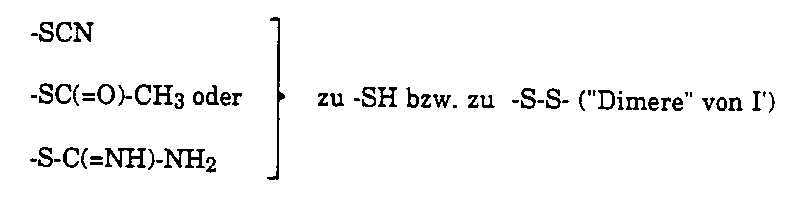

a) mittels H⁺/H₂O
   in polaren Lösungsmitteln (z.B. EtOH, H₂O, etc.)
   oder
b) mittels HO⁻/R⁵O⁻ (R⁵ = Me, Et, t-Butyl, etc.)
   in polaren Lösungsmitteln (EtOH, H₂O, etc.) durchgeführt T = 0-100°C, z.B. ca. 20°C

### β) Reduktion

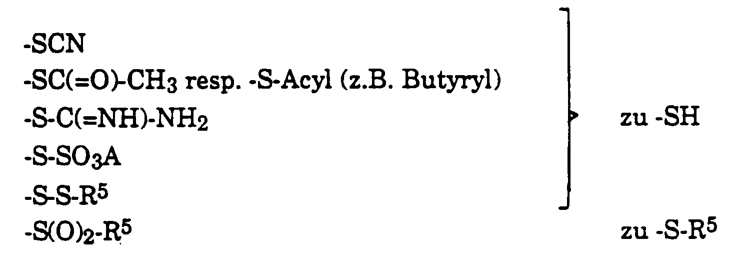

### Reduktionsmittel

z.B. NaBH₄, LiAlH₄, NaBH₃CN
Sn/HCl, Zn/CH₃COOH, Na₂S₂O₃
Phosphine (z.B. Tributyl(phosphin)
aber auch Na/NH₃ oder
H₂ kat. (Kat. = z.B. Pd, Pt, Rh, etc.)
vorzugsweise in polaren Lösungsmitteln wie H₂O/EtOH,
Essigsäure, Aether, MTBE, Tetrahydrofuran, etc.
T = ca. -80°C bis ca. 100°C, z.B. 20°C

### γ) Oxidation

-SH zu -S-S- ("Dimeres" von I')
mittels J₂ oder H₂O₂ in polaren Lösungsmitteln (z.B. EtOH, H₂O)
T=0-100°C

### δ) Substitution

nämlich am S-Atom in 3-Stellung der Verbindung I' durch Austausch des an dieses S gebundenen Seitenkettenrestes, z.B. durch den neuen Seitenkettenrest R⁴ oder SR⁵ mittels geeigneter Reagenzien; sie kann erfolgen z.B. gemäss

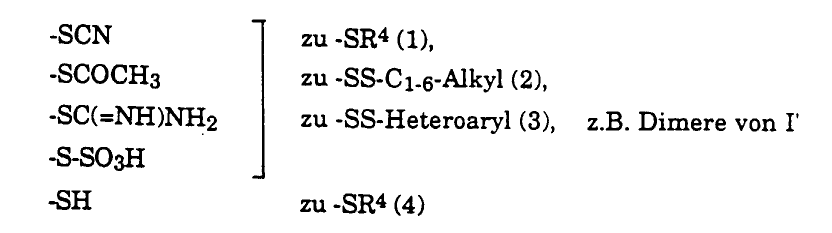

### mittels folgender Reagenzien

(1) Hal-R⁴, z.B. CH₃J, Cl(CH)₂COCH₃, BrCH₂COOH, etc.,
(2) A-S-Alkyl, z.B. A-SCH₃,
(3) A-S-Heteroaryl, z.B. 3-Mercaptothiophen, Furfurylmercaptan, 3-Mercaptofuran, etc.,
(4) HalR⁴.

Aus dieser Zusammenstellung ist ersichtlich, dass das Radikal C der Formel I die durch B und S-R definierten Reste umfasst.

### Bedingungen:

vorzugsweise in polaren Lösungsmitteln, wie H₂O, Aethanol, Aethern, Säuren wie Essigsäure, gegebenenfalls unter Verwendung von Basen, wie den üblichen Hydroxiden, Carbonaten, Bicarbonaten, Alkylaten, Acetaten, tert.Aminen, etc. bei Temperaturen von ca. 0° bis ca. 10°C.

### Aufarbeitung

Die Aufarbeitung erfolgt vorzugsweise durch Extraktion: Ausschütteln zwischen Wasser oder allenfalls verdünnter Säure und einem organischen Lösungsmittel, wie Aether, MTBE, CH₂Cl₂, Hexan, Pentan, Toluol, etc., Abtrennung der organischen Phasen und Einengen (Abdampfen) des organischen Lösungsmittels.

### Reinigung

Diese geschieht vorzugsweise durch Destillation oder allenfalls durch Chromatographie.

Die Verbindungen I sind grösstenteils bekannte, teilweise in der Natur vorkommende Aromastoffe, z.B. Fleischaromastoffe.

Beispiele solcher Stoffe sind die in den untenstehenden Beispielen exemplifizierten Mercaptane und Disulfide.

Als weitere Beispiele können genannt werden:

### Subst. Alkylderivate

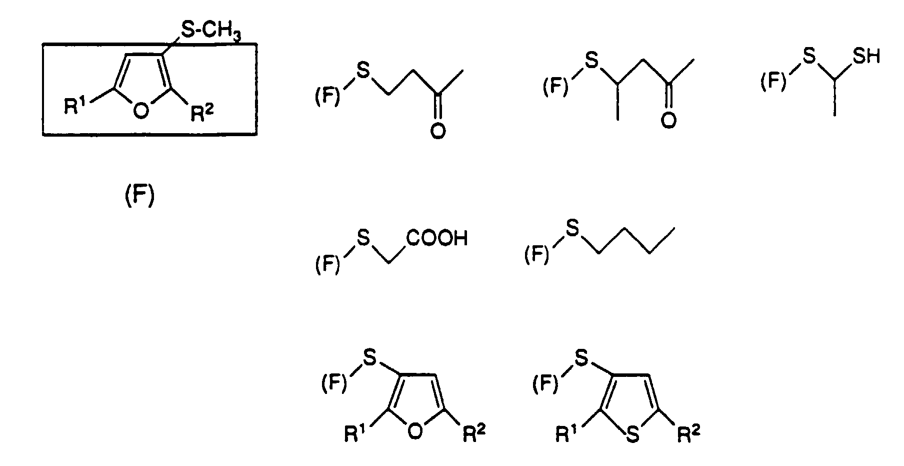

### Acylderivate

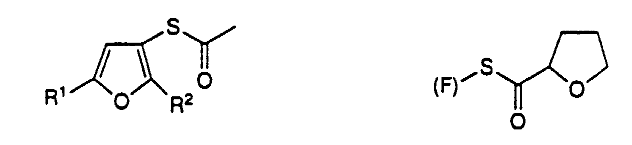

### S-Alkylderivate

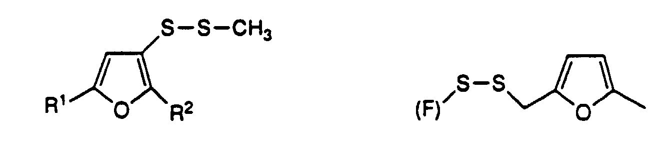

### S-Aryle bzw. Heteroaryle

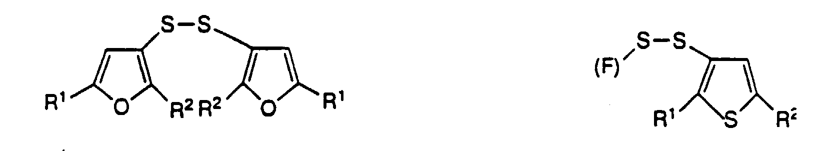

Die obigen Mono-S-Verbindungen sind durch die erfindungsgemässe Primärreaktion zugänglich.

Die obigen Disulfide sind insbesondere durch eine der Folgereaktionen α) bis γ) zugänglich, wobei dabei die Substitution im Vordergrund steht.

Die Formel I umfasst aber definitionsgemäss auch die Verbindungen I‴, nämlich die Verbindungen 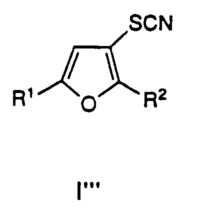 worin R¹ und R²H, CH₃ oder C₂H₅ bedeuten und R¹=R²≠H.

Diese Verbindungen sind neue Zwischenprodukte zur Herstellung der Aromastoffe.

Bevorzugt sind im Falle der Aromastoffe I insbesondere die naturidentischen Verbindungen.

### Beispiel 1

### 3,3'-Bis-(2,5-dimethylfuryl)-disulfid

### a) 2,5-Dimethyl-3-thioacetylfuran

Zu einem Gemisch von 11,42 g Thioessigsäure in 50 ml Methylenchlorid und 100 ml 2N Schwefelsäure tropft man unter Rühren innert 15 Min. 5,61 g 5-Hydroxyhex-3-in-2-on [hergestellt nach A.F.Thomas et al., Tet.Let. 27, 505, (1986)] in 50 ml Methylenchlorid. Nach 2 Stunden Stehenlassen bei Raumtemperatur wird auf 40°C erhitzt und noch 4 Std. bei 40°C weitergerührt. Das Reaktionsgemisch wird auf 100 ml H₂O gegossen und 2 x mit 100 ml Methylenchlorid extrahiert. Die über MgS0₄ getrockneten organischen Phasen werden eingeengt und am HV fraktioniert destilliert (65°C/ 0,2mbar). Man erhält 5,7 g (67% der Theorie) einer gelben Flüssigkeit.
NMR (CDCl₃) 2,22 ppm (s/3H); 2,26 (s/3H); 2,38 (s/3H); 5,89 (s/1H).

### b) 3,3'-Bis-(2,5-dimethylfuryl)-disulfid

2 g des oben erhaltenen Thioacetates werden in 25 ml Methanol gelöst und mit 250 mg Natriumcarbonat versetzt. Nach 3 Stunden Rühren bei 50°C wird das Reaktionsgemisch zwischen Wasser und Aether verteilt und die organischen Phasen über Magnesiumsulfat getrocknet und eingeengt. Die erhaltenen 1,3 g Rohprodukt werden bei 100°C / 0,13mbar destilliert.
NMR (CDCl₃) 2,09 ppm (s/6H); 2,24 (s/6H); 5,97 (s/2H).

### Beispiel 2

### 3,3'-Bis-(2-methylfuryl)-disulfid

### a) 2-Methyl-3-thioacetylfuran

Es wird gemäss Beispiel 1a verfahren, jedoch wird statt 5-Hydroxyhex-3-in-2-on 4-Hydroxy-2-pentinaldiethylacetal [hergestellt nach R.G.Jones und M.J.Mann, J.Am.Chem.Soc. 75, 4048, (1953)] eingesetzt. Man erhält in 12%iger Ausbeute eine gelbe Flüssigkeit (Sdp 35°C / 0,2mbar).
NMR (CDCl₃) : 2,3 ppm (s/3H); 2,4 (s/3H); 6,35 (d/1H); 7,4 (d/1H).

### b) 3,3'-Bis-(2-methylfuryl)-disulfid

Das oben erhaltene Thioacetat wird gemäss Beispiel 1b umgesetzt. Man erhält das gewünschte Produkt Sdp. 77°C / 0,4mbar.
NMR (CDCl₃) 2,1 ppm (s/6H); 6,38 (s/2H); 7,28 (s/2H).

### Beispiel 3

### 3-3'-Bis-(2,5-dimethylfuryl)-disulfid

### a) 3-(2,5-Dimethylfuryl)-thiocyanat

Zu einer Lösung von 115,3 g Natriumrhodanid in 1300 ml 2N Schwefelsäure werden 1300 ml Pentan zugegeben. Dann tropft man unter Rühren innert 120 Minuten 145 g 5-Hydroxyhex-3-in-2-on [hergestellt nach A.F.Thomas et al., Tet.Let. 27, 505, (1986)] zu. Die Phasen werden getrennt und die wässrige Phase wird noch 2 x mit 1000 ml Pentan extrahiert. Die vereinigten organischen Phasen werden mit 250 ml gesättigter NaCl-Lösung gewaschen, über MgS0₄ getrocknet, eingeengt und am HV fraktioniert destilliert (53°C / 0,14mbar). Man erhält 94,8 g (48% der Theorie) einer gelben Flüssigkeit.
NMR (CDCl₃) : 2,22 ppm (s/3H); 2,38 (s/3H); 6,06 (s/1H).

### b) 3-3'-Bis-(2,5-dimethylfuryl)-disulfid

7,6 g Natriumhydroxid werden in 200 ml H₂O gelöst und 14,6 g des oben erhaltenen Thiocyanates werden unter Rühren innert 30 Minuten zugetropft. Nach 90 Minuten Raumtemperatur wird das Reaktionsgemisch zwischen Wasser und Pentan verteilt, die organischen Phasen über MgS0₄ getrocknet, eingeengt und am HV bei 100°C / 0,13mbar destilliert. Man erhält 9,2 g (76% Ausbeute) einer gelben Flüssigkeit.
NMR-Spektrum siehe Beispiel lb.

### Beispiel 4

### 3-Methylthio-2,5-dimethylfuran

Zu einer Lösung von 13,7 g Natriumhydroxid und 35,6 g Methyljodid in Methanol tropft man unter Rühren innert 40 Minuten 3-(2,5-Dimethylfuryl)-thiocyanat (hergestellt gemäss Beispiel 3a) in 50 ml Methanol. Dann wird das Reaktionsgemisch auf 500 ml H₂0 geworfen und 3 x mit 700 ml Methyl-tert-butylether extrahiert. Die über MgS0₄ getrockneten organischen Phasen werden eingeengt und am Wasserstrahlvakuum bei 71°C / 19mbar destilliert. So erhält man 22,8 g (70% der Theorie) eines klaren Oels.
NMR(CDCl₃); 2,22 ppm (s/3H); 2,25 (s/3H); 2,28 (s/3H); 5,94 (s/1H).

### Beispiel 5

### 2,5-Dimethyl-3-furanthiol (eine speziell bevorzugte Verbindung)

126,8 g Kaliumcarbonat und 12,7 g Natriumdithionit werden in 400 ml Wasser gelöst, dann werden nacheinander unter Rühren 200 ml 2-Mercaptoethanol und 8,5 g 3-(2,5-Dimethylfuryl)-thiocyanat (hergestellt gemäss Beispiel 3a) zugetropft. Nach 30 Min. Stehenlassen bei Raumtemperatur wird das Reaktionsgemisch mit 200 ml konz. Salzsäure langsam auf pH 2 gebracht und die wässerige Phase 3 x mit 300 ml Pentan extrahiert. Die über MgS0₄ getrockneten organischen Phasen werden eingeengt und am Wasserstrahlvakuum bei 80°C /13mbar destilliert. Man erhält 4,5 g (57% Ausbeute) einer roten Flüssigkeit.
NMR (CDCl₃) : 2,22 ppm (s/3H); 2,27 (s/3H); 2,62 (d/1H); 5,88 (s/1H).

### Beispiel 6

### 2-Methyl-3-furanthiol (eine speziell bevorzugte Verbindung)

### a) 3-(2-Methylfuryl)-thiocyanat

Es wird gemäss Beispiel 3a gearbeitet, jedoch statt S-Hydroxyhex-3-in-2-on, 4-Hydroxy-2-pentinaldiethylacetal [hergestellt nach R.G.Jones und M.J.Mann, J.Am.Chem.Soc. 75, 4048 (1953)] eingesetzt. Man erhält nach fraktioniertem Destillieren am HV bei 51°C / 0,6mbar in 80% Ausbeute 3-(2-Methylfuryl)-thiocyanat.
NMR (CDCl₃) : 2,42 ppm (s/3H); 6,5 (d/1H); 7,37 (d/1H).

### b) 2-Methyl-3-furanthiol

Zu einer Lösung von 2,1 g 3-(2-Methylfuryl)-thiocyanat in 20 ml Methanol werden 0,6 g Natriumborhydrid zugegeben und auf Rückflusstemperatur erwärmt. Nach 3 Stunden Rühren unter Rückfluss werden nochmals 0,6 g Natriumborhydrid zugegeben und es wird 15 Minuten weitergerührt. Dann wird das Reaktionsgemisch auf 50 ml Wasser gegossen und 3 x mit 50 ml Methyl-tert-butylether extrahiert. Die vereinigten organischen Phasen werden über MgS0₄ getrocknet und eingeengt. Die erhaltenen 1,6 g Rohprodukt werden bei 140°C / Normaldruck destilliert.
NMR (CDCl₃) 2,33 ppm (s/3H); 2,66 (d/1H); 6,3 (d/1H); 7,25 (d/1H).

### Beispiel 7

### Methyl 2-methyl-3-furyl-disulfid

Zu einer Lösung von 4,3 g Natriummethylmercaptid in 100 ml Wasser tropft man unter Rühren innert 25 Minuten 3-(2-Methylfuryl)thiocyanat (hergestellt gemäss Beispiel 6a). Nach 30 Minuten Rühren bei Raumtemperatur werden 100 ml Pentan zugegeben, und die wässerige Phase wird von der organischen Phase getrennt. Die wässerige Phase wird noch 2 x mit 100 ml Pentan extrahiert. Die vereinigten organischen Phasen werden über MgS0₄ getrocknet, eingeengt und am HV fraktioniert destilliert (37°C 0,48mbar). Man erhält das gewünschte Produkt.
NMR (CDCl₃); 2,4 ppm (s/3H); 2,45 (s/3H); 6,44 (d/1H); 7,28 (d/1H).

### Beispiel 8

### 3-n-Butylthio-2,5-dimethylfuran

6,2 ml Triethylamin und 4,02 g n-Butylmercaptan tropft man gleichzeitig unter Rühren zu einer Lösung von 5 g S-Hydroxyhex-3-in-2-on [hergestellt nach A.F.Thomas et al., Tet.Let. 27, 505, (1986)] in 40 ml Methyl-tert-butylether (MTBE). Nach 60 Minuten Raumtemperatur werden 70 ml 5N Schwefelsäure zugegeben und 60 Minuten weitergerührt. Dann werden die Phasen getrennt, die wässerige Phase wird mit 2 x 50 ml MTBE extrahiert und die organische Phase nacheinander mit Wasser, gesättigter NaHCO₃ Lösung und gesättigter NaCl Lösung gewaschen. Die über MgS0₄ getrockneten organischen Phasen werden eingeengt und am HV fraktioniert destilliert (63°C / 0,25mbar). Man erhält 6,34 g (77% Ausbeute) des gewünschten Produkts.
NMR (CDCl₃): 0,88 ppm (t/3H); 1,46 (Multiplett / 4H); 2,22 (s/3H); 2,28 (s/3H); 2,58 (t/2H); 5,6 (s/1H).

### Beispiel 9

### 3-Methylthio-2,5-dimethylfuran

Es wird gemäss Beispiel 8 verfahren, jedoch wird statt n-Butylmercaptan Methylmercaptan eingesetzt. So erhält man nach fraktioniertem Destillieren am Wasserstrahlvakuum (58°C/ 22mbar) 65% Ausbeute eines klaren Oels. Spektren: siehe Beispiel 4.

### Beispiel 10

### 3-Methylthio-2,5-dimethylfuran

Dasselbe Produkt wie in Beispiel 9 beschrieben erhält man auch, wenn man 5-Hydroxyhex-3-in-2-on zu einem Gemisch von Natriummethanthiolat in Wasser und Methyl-tert-butyläther zutropft und darauf - wie in Beispiel - 8 mit Schwefelsäure behandelt und aufarbeitet.

### Beispiel 11

### 3-3'-Bis-(2,5-dimethylfuryl)-disulfid

Zu einem Gemisch von 6,6 g Natriumhydrogensulfid-monohydrat in 20 ml Puffer pH 9 (Borax/Borsäure) und 30 ml Ether tropft man unter Rühren 5 g S-Hydroxyhex-3-in-2-on in 25 ml Ether. Nach 3 Stunden Rühren wird angesäuert und eine Stunde weitergerührt. Die organische Phase wird abgetrennt, eingeengt und in Hexan über Kieselgel chromatographiert. Die eingeengten Kopffraktionen ergeben reines Produkt, welches mit dem Produkt aus Beispiel 1a identisch ist.

## Patentansprüche

1. Verfahren zur Herstellung von Verbindungen der Formel 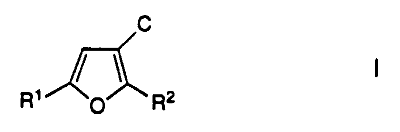
worin R¹ und R² bedeuten: Wasserstoff, C₁₋₆-Alkyl, C₂₋₆-Alkenyl, und
C einen schwefelhaltigen Rest darstellt,
dadurch gekennzeichnet, dass man eine Verbindung 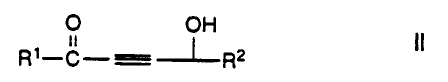 oder ein Acetal oder Ketal davon, mittels einer, mindestens ein nukleophiles Schwefelatom enthaltenen Verbindung
AB III
worin
A = H⁺, NH₄⁺, N(C₁₋₆Alk)₃H⁺, ein Alkalimetallkation, Erdalkalimetallkation,
B = R⁴-S⁻, SCN⁻, AS₂O₃⁻, H₂N-C(=S)NH⁻, R⁵-S(=O)O⁻, R⁵-S-S und
R⁴ = H, C₁₋₆-Alkyl, C₂₋₆-Alkenyl, C₂₋₆-Alkinyl, einen durch Halogen, OH, SH, C=O oder einen C₁₋₆-Carbonsäurerest oder ein C₁₋₆-Alkylcarbonsäurederivat substituiertes C₁₋₆-Alkyl
C₁₋₆-Acyl, gegebenenfalls substituiertes Acyl,
R⁵ = C₁₋₆-Alkyl, Aryl, Heteroaryl,
cyclisiert, und das primäre Reaktionsprodukt der Formel 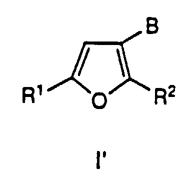 gegebenenfalls durch eine Sekundärreaktion in ein Folgeprodukt der Formel 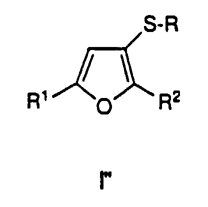 worin R = R⁴; oder SR⁵, worin SR⁵ gegebenenfalls mit Halogen, Hydroxy, SH, Carbonyl, Carboxyl funktionalisiertes S-C₁₋₆-Alkyl, S-Aryl oder S-Heteroaryl darstellt,
überführt.

2. Verfahren nach Anspruch 1, dadurch gekennzeichnet, dass man die Cyclisierung unter sauren Bedingungen durchführt, wobei diese saure Cyclisierung gegebenenfalls durch eine basische Vorbehandlung des Gemisches der Reaktionspartner II und III eingeleitet wird.

3. Verfahren nach Anspruch 1 oder 2, dadurch gekennzeichnet, dass die Sekundärreaktion eine Hydrolyse, eine Reduktion, eine Oxidation oder eine Substitution darstellt.

4. Verfahren nach einem der Ansprüche 1 oder 2, dadurch gekennzeichnet, dass sind:
R¹, R² H oder C₁₋₃-Alkyl,
R³ CH₃ oder C₂H₅,
R⁴ H, C₁₋₆-Akyl, C₁₋₆-Acyl,
R⁵ CH₃ oder Phenyl,
R H, CH₃ -S-CH₃, gegebenenfalls substituiertes S-Heteroaryl, wie 3-S-Furyl, 3-S-Thio-furyl, Thio-furfuryl,
B SCN⁻, AS₂O₃⁻, CH₃S⁻, CH₃COS⁻.

5. Verfahren nach Anspruch 4, dadurch gekennzeichnet, dass R H, CH₃ oder CH₃S⁻ bedeutet.

6. Verfahren nach Anspruch 4, dadurch gekennzeichnet, dass R Wasserstoff und R¹ Wasserstoff oder Methyl und R² Methyl sind.

7. Verbindungen der Formel 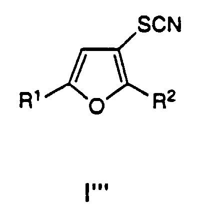 wobei R^{1'} und R^{2'} H, CH₃ oder C₂H₅ bedeuten und R^{1'} = R^{2'} ≠ H.

## Claims

1. A process for the manufacture of compounds of the formula 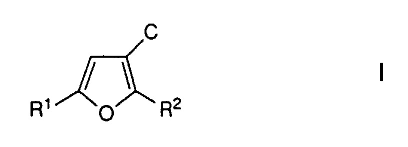
wherein R¹ and R² signify hydrogen, C₁₋₆-alkyl, C₂₋₆-alkenyl and
C represents a sulphur-containing residue,
characterized by cyclizing a compound 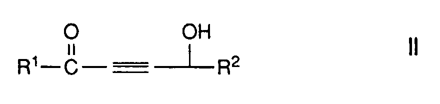 or an acetal or ketal thereof using a compound III which contains at least one nucleophilic sulphur atom
AB III
wherein
A = H⁺, NH₄⁺, N(C₁₋₆Alk)₃H⁺, an alkali metal cation, alkaline earth metal cation,
B = R⁴-S⁻, SCN⁻, AS₂O₃⁻, H₂N-C(=S)NH⁻, R⁵-S(=O)O⁻, R⁵-S-S and
R⁴ = H, C₁₋₆-alkyl, C₂₋₆-alkenyl, C₂₋₆-alkynyl, C₁₋₆-alkyl substituted by; halogen, OH, SH, C=O or a C₁₋₆-carboxylic acid residue or a C₁₋₆-alkylcarboxylic acid derivative;
C₁₋₆-acyl, optionally substituted acyl,
R⁵ = C₁₋₆-alkyl, aryl, heteroaryl,
and, if desired, converting the primary reaction product of the formula 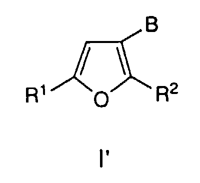 by a secondary reaction into a subsequent product of the formula 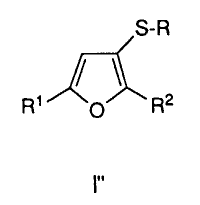 wherein R = R⁴; or SR⁵ in which SR⁵ represents S-C₁₋₆-alkyl optionally functionalized with halogen, hydroxy, SH, carbonyl, carboxyl, S-aryl or S-heteroaryl.

2. A process according to claim 1, characterized in that the cyclization is carried out under acidic conditions, whereby this acidic cyclization is optionally preceeded by a basic pretreatment of the mixture of reaction partners II and III.

3. A process according to claim 1 or 2, characterized in that the secondary reaction is a hydrolysis, a reduction, an oxidation or a substitution.

4. A process according to either claim 1 or 2, characterized in that:
R¹, R² are H or C₁₋₃-alkyl,
R³ is CH₃ or C₂H₅,
R⁴ is H, C₁₋₆-alkyl or C₁₋₆-acyl,
R⁵ is CH₃ or phenyl,
R is H, CH₃ -S-CH₃, optionally substituted S-heteroaryl, such as 3-S-furyl, 3-S-thio-furyl, thio-furfuryl,
B is SCN⁻, AS₂O₃⁻, CH₃S⁻, CH₃COS⁻.

5. A process according to claim 4, characterized in that R signifies H, CH₃ or CH₃S⁻.

6. A process according to claim 4, characterized in that R is hydrogen and R¹ is hydrogen or methyl and R² is methyl.

7. Compounds of the formula 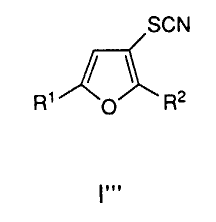 in which R^{1'} and R^{2'} signify H, CH₃ or C₂H₅ and R^{1'} = R^{2'} ≠ H.

## Revendications

1. Procédé de préparation de composés de formule 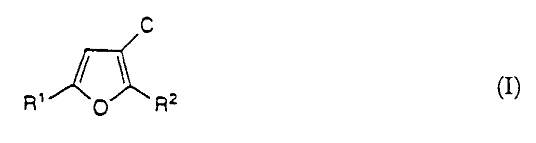
dans laquelle R¹ et R² représentent : un hydrogène, un alkyle en C₁ à C₆, un alcényle en C₂ à C₆, et
C représente un radical contenant du soufre,
caractérisé en ce qu'on cyclise un composé 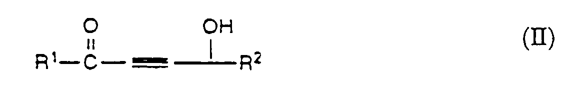 ou un de ses acétals ou cétals, au moyen d'un composé contenant au moins un atome de soufre nucléophile
AB (III)
dans lequel
A = H⁺, NH₄⁺, N(alc en C₁ à C₆)₃H⁺, un cation de métal alcalin, un cation de métal alcalino-terreux,
B = R⁴-S⁻, SCN⁻, AS₂O₃⁻, H₂N-C(=S)NH⁻, R⁵-S(=O)O⁻, R⁵-S-S et
R⁴ = H, alkyle en C₁ à C₆, alcényle en C₂ à C₆, alcynyle en C₂ à C₆; un alkyle en C₁ à C₆ substitué par un halogène, OH, SH, C=O ou un radical acide carboxylique en C₁ à C₆ ou un dérivé d'acide alkyle en C₁ à C₆-carboxylique;
un acyle en C₁ à C₆, un acyle éventuellement substitué,
R⁵ = alkyle en C₁ à C₆, aryle, hétéroaryle,
et en ce qu'on transforme le produit de réaction primaire de formule 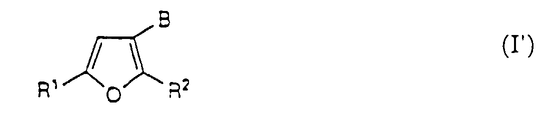 le cas échéant par une réaction secondaire pour donner un dérivé de formule 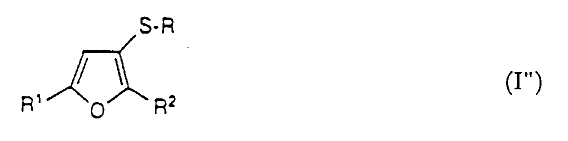 dans laquelle R = R⁴ ; ou SR⁵, où SR⁵ représente un S-alkyle en C₁ à C₆, un S-aryle ou un S-hétéroaryle portant éventuellement des fonctions halogène, hydroxy, SH, carbonyle, carboxyle.

2. Procédé selon la revendication 1, caractérisé en ce qu'on procède à la cyclisation dans des conditions acides, la cyclisation acide étant éventuellement introduite par un prétraitement basique du mélange des réactifs II et III.

3. Procédé selon la revendication 1 ou 2, caractérisé en ce que la réaction secondaire représente une hydrolyse, une réduction, une oxydation ou une substitution.

4. Procédé selon une des revendications 1 ou 2, caractérisé en ce que les symboles ci-dessous ont les significations suivantes :
R¹, R² H ou alkyle en C₁ à C₃,
R³ CH₃ ou C₂H₅,
R⁴ H, alkyle en C₁ à C₆, acyle en C₁ à C₆,
R⁵ CH₃ ou phényle,
R H, CH₃, -S-CH₃, S-hétéroaryle éventuellement substitué, comme 3-S-furyle, 3-S-thio-furyle, thio-furfuryle,
B SCN⁻, AS₂O₃⁻, CH₃S⁻, CH₃COS⁻.

5. Procédé selon la revendication 4, caractérisé en ce que R représente H, CH₃ ou CH₃S⁻.

6. Procédé selon la revendication 4, caractérisé en ce que R représente un hydrogène et en ce que R¹ représente un hydrogène ou un méthyle et R² représente un méthyle.

7. Composés de formule 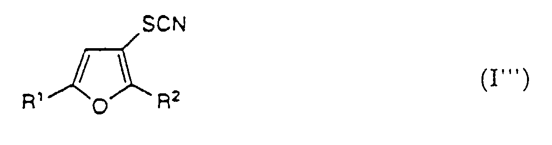 dans laquelle R^{1'} et R^{2'} représentent H, CH₃ ou C₂H₅ et R^{1'} = R^{2'} ≠ H.
